# EUROPEAN PATENT SPECIFICATION

(11) **EP 1 207 917 B1**
(45) Date of publication and mention of the grant of the patent: **28.05.2003**
(21) Application number: 00956694.4
(22) Date of filing: 01.09.2000
(51) Int. Cl.: A61L 27/34, A61L 27/58

(54) **SEALANT FOR VASCULAR PROSTHESES**
DICHTUNGSMITTEL FÜR GEFÄSSPROTHESEN
MATERIAU D'ETANCHEITE POUR PROTHESES VASCULAIRES

(30) Priority: 03.09.1999 GB 9920732
(43) Date of publication of application: 29.05.2002
(73) Proprietor: VASCUTEK LIMITED, Renfrewshire, Scotland PA4 9RR (GB)
(72) Inventor: KELSO, Karen, Anne, Kilmarnock KA3 6BJ (GB)
(74) Representative: Ouzman, Beverley Nicola Claire
(86) International application number: GB0003343
(87) International publication number: WO01017571

(56) References cited:
- EP-A- 0 183 365
- EP-A- 0 633 032
- EP-A- 0 742 020
- GB-A- 854 715
- US-A- 4 902 290
- US-A- 5 292 362
- US-A- 5 851 229

## Description

The present invention relates to a non-gelatine based coating or sealant for porous vascular prostheses, and to a method of making that coating or sealant.

Porous vascular prostheses constructed from textiles (such as polyester) are normally woven or knitted and ultimately rely on host tissue penetrating into the spaces between the yarns. To function in the long term the prostheses must, therefore, acquire porosity whilst at implant bleeding through the prosthesis wall must be prevented or at least limited to an acceptable level.

In the past this dilemma has been resolved by soaking a porous textile-based prosthesis in the patient's blood which then clots to form a seal. This pre-clotting technique is time consuming, exposes the prosthesis to potential contamination and may be ineffective in patients with reduced clotting ability (either reduced spontaneous blood clotting or through administration of anti-platelet or anti-thrombotic medication).

More recently, vascular prostheses have been pre-sealed with a variety of bioresorbable materials. The sealants tried to date have tended to be protein based, such as collagen, gelatin or albumen. Cross-linkers such as glutaraldehyde, formaldehyde, carbodiiamide or isocyanates have been used to render the proteins insoluble and mention may be made of EP-B-0,183,365; US-A-4,747,848 and US-A-4,902,290, all of which describe the preparation of cross-linked gelatin-based sealants. Hydrolysis or enzymatic attack in the host tissue has then gradually degraded or removed the sealant from the textile to permit the necessary tissue ingrowth.

The prior art protein based sealants are derived from animal or human sources, which creates the potential for transmission of infection. This has been especially of concern following transmission of BSE to humans which has greatly elevated public concern over the safety of animal derived implants. Additionally, although some materials, such as gelatin, are produced in large commercial quantities and blended to give high lot-to-lot consistency, manufacture from natural raw materials always has the potential for variability which creates uncertainty regarding performance of the graft.

US8 1 229 describes a bioresorbable sealant composition including at least two polysaccharides, which may be cross-linked. The present invention relates to a bioresorbable sealant which is not animal derived but is based on cross-linked dextran. Dextran is produced by a fermentation process using *Leuconostoc mesenteroides* bacteria growing on a sugar-based energy source, such as sucrose. Partial hydrolysis of the fermentation product yields dextrans of defined molecular weight. These have been used widely as plasma substitutes with a typical molecular weight of 40,000.

Dextrans of this molecular weight are freely water-soluble. To form a useful graft sealant, the dextrans must be rendered insoluble. However, dextrans are not easily cross-linked as they have limited reactive sites to form intermolecular bonds. The available groups are almost exclusively hydroxyl (OH) groups.

British Patent No 854,715 describes the formation of a dextran-based polymer by using epichlorohydrin. However the epichlorohydrin-based approach forms very stable cross-links so that the resultant polymer is resistant to both enzymatic and hydrolytic attack and does not biodegrade. Epichlorohydrin cross-linked dextran is, therefore, unsuitable as a vascular graft sealant as it is not bioresorbable and would not permit tissue ingrowth within the timescale required. EP-B-0,183,365 and US-A-4,747,848 both describe a gelatin-based sealant in which the time-scale of reabsorption is controllable.

To overcome this problem, a novel dextran-based polymer has been produced which is bioresorbable through hydrolysis in the time scale of interest.

The present invention provides a bioresorbable sealant composition comprising a polymer formed by reaction between dextran, formaldehyde and urea. Whilst the dextran polymer product is insoluble, the polymer is formed with bonds that are sufficiently labile to permit resorption at an appropriate rate for tissue ingrowth. Furthermore, when the cross-linked polymer breaks down, it does so into simple products all of which have a low molecular weight and which are easy for the body to dispose of.

The term "dextran" as used herein includes naturally occurring dextran (especially that obtained through fermentation of micro-organisms such as *Leuconostoc sp.)* as well as hydrophilic hydroxyl group-containing derivatives of dextran, for example partially depolymerized dextran, dextran glycerine glycoside or hydrodextran. Also included are modified forms of dextran containing other reactive groups, for example carboxyl, sulphonic, sulphate, amino or substituted amino groups. Mention may be made of carboxymethyldextran and dextran sulphate as examples of modified dextran. Mixtures of different dextrans (as defined herein) may of course also be used, where appropriate.

The polymer described herein is formed in water or an aqueous-based solvent. It is therefore essential that the dextran selected as the initial reactant should be water-soluble or in the form of swollen particles.

Dextrans having a molecular weight of 10,000 to 100,000, in particular 20,000 to 80,000, especially 30,000 to 60,000 may be used. Preferably the dextran used in the invention has a typical molecular weight of about 40,000.

Viewed from one aspect, therefore, the present invention provides a method of forming polymerised dextran for use as a biodegradable coating for a prosthetic graft, said method comprising:
a) exposing a water-based solution of dextran to 2 to 25 (weight %) of urea and allowing the urea to enter into solution to form a mixture;
b) exposing the mixture of step a) to formaldehyde;
c) heating the mixture of step b) at temperatures between 20 to 250°C for a time sufficient to allow polymerisation to occur.

The formaldehyde is conveniently added in the form of formalin (a 37% aqueous solution of formaldehyde hydrate). Alternatively, it would be possible to bubble formaldehyde gas through the mixture of step (a) to achieve the required reaction. The quantity of formaldehyde required may be determined stochiometrically having regard to the amount of urea added in step (a). We have found that an amount of formaldehyde equivalent to 50 to 100% (by weight) with reference to the amount of urea achieves the required result, with 70 to 80% (by weight) being preferred. Usually a time period of from five to 60 minutes is sufficient to permit the cross-linking reaction to occur.

In a further aspect, the present invention provides a method of producing a non-porous graft by impregnating or coating a flexible material with a mixture of dextran, urea and formaldehyde, and incubating said impregnated material at temperatures of from 20°C to 250°C for a time sufficient to facilitate cross-linking of said dextran. The flexible material to be impregnated or coated will usually be a macroporous (eg a knitted or woven) fabric. However, non-porous or microporous materials may likewise be coated, with the sealant reducing blood loss after suturing.

Preferably the temperature selected is from 30°C to 200°C, for example is from 45°C to 160°C.

The flexible porous material to be treated by the present invention may be of any conventional type or construction. Particular mention may be made of polyester (e.g. DACRON™) knitted or woven fabric and also of PTFE-based materials. Additionally, expanded PTFE may be coated as described since, although the material itself is non-porous, porosity will be introduced when the graft is stitched into place by the surgeon. The graft may be simply immersed in the reaction mixture or may be selectively dipped therein (for example the graft may be placed on a mandrel and "rolled" over the surface of the reaction mixture to coat the external surface only). Optionally pressure may be used to ensure penetration of the reaction mixture into the interstices of a porous graft.

In a further aspect, the invention also provides a prosthetic graft impregnated or coated with the bioresorbable sealant of the invention. The graft may be, for example, a knitted polyester graft.

To prevent the sealant from drying out on the graft and becoming brittle in storage it is advantageous to plasticise the treated graft with a biocompatible agent such as glycerol. This is preferably achieved by treating the sealed grafts with glycerol after cross-linking of the dextran. Excess glycerol may be removed by alcohol rinsing. Suitable alcohols include ethanol, methanol and propanol, but other alcohols may also be used.

As described above, the treated graft may be plasticised. Alternatively, or additionally, the graft may undergo a separate sterilisation step, for example by exposure to γ-irradiation. Sterilisation may not be required if the graft and coating have been formed in sterile conditions.

The primary mechanism of polymerisation involves a urea/formaldehyde condensation reaction, where the application of high temperature and water encourages polymerisation of the dextran reactant. Subsequent condensation reactions involve primary hydroxyl groups present on the dextran molecule. Due to the small levels of urea and formaldehyde required to cause the reactions it was believed the process needed only short urea-formaldehyde condensate links to give good cross-linking parameters. Subsequently formed bonds were identified as reactive ether bonds which were subject to hydrolytic degradation. Various forms of analysis such as NMR and FTIR have confirmed that the degradation products are of low molecular weight and likely to comprise sugar units, urea, formaldehyde and small complexes of the latter components. It is of course possible to modify the hydroxyl groups available on the dextran for reaction (see for example EP-B-0,183,365).

The use of dextran sulphate is desirable since the cross-linked polymer so produced contains sulphate groups available for binding, for example, to the heparin binding site of fibroblast growth factor. Fibroblast growth factors form a large family of structurally related, multifunctional proteins that regulate various biological responses and have been implicated in many developmental and regenerative events including axial organisation, mesodermal patterning, keratinocyte organisation and brain development. These compounds mediate cellular functions by binding to transmembrane fibroblast growth factor receptors, which are protein tyrosine kinases. Fibroblast growth factor receptors are activated by oligomerisation and both this activation and fibroblast growth factor stimulated biological responses, require the presence of "heparin-like" molecules as well as fibroblast growth factor.

Heparins are linear polysaccharide chains; they are typically heterogeneously sulphated on alternating L-iduronic and D-glycosamino sugars. A review of the fibroblast growth factor molecular complexes associated with heparin-like sugars has recently been undertaken (DiGabriele et al., 1998; ISSN 0028-0836). Heparin sulphates, the N-sulphated polysaccharide components of proteoglycans, are common constituents of cell surfaces and the extracellular matrix. The heparin sulphate polysaccharide chain has a unique molecular design in which the clusters of N and O-sulphated sugar residues, separated by regions of low sulphation, determine specific protein binding properties. Current data indicates that relatively long specific binding sequences of heparin sulphate may induce a conformational change in basic fibroblast growth factor, exposing a site on the protein that is recognised by signal transducing receptors. There are also suggestions that the core protein of plasma membrane heparin sulphate-proteoglycans may participate in the cell signalling process (Gallagher, 1994; ISSN 0939-4974).

The heparin sulphate chains are attached to various protein cores, which determine the location of the proteoglycan in the cell membrane and extracellular matrix. The diverse functions of heparin sulphate, which range from the control of blood coagulation to the regulation of cell growth and adhesion, depend on the capacity of the chains to activate protein ligands, such as antithrombin III and members of the fibroblast growth factor family. These properties are currently being exploited in the development of synthetic heparin sulphates as anticoagulants and promoters of wound healing. Conversely organic mimics of growth factor-activating saccharides could possibly be designed to suppress tumour growth and prevent restenosis after coronary vessel angioplasty (Stringer and Gallagher, 1997; ISSN 1357-2725). Earlier researchers had also reported on the theory that fibroblast growth factor receptors might be directly activated by a much wider range of ligands, including heparin sulphate proteoglycans and neural cell adhesion molecules as well as related sulphonated compounds (Green et al., 1996; ISSN 0265-9247). As early as 1994 research groups were investigating areas which would aid the design of synthetic sulphonated oligosaccharides aimed at improving the bioavailability of fibroblast growth factor when administered *in vivo* as a therapeutic agent (Coltrini et al., 1994; ISSN 0264-6021). Thus, Belford et al (1993) in Journal of Cellular Physiology 157 : 184-189 describes the ability of several animal, plant and bacterial derived polyanions as well as synthetic polyanions to compete with heparin for the binding of acidic fibroblast growth factor and correlates this with their ability to potentiate the mitogenic and neurotrophic actions of this factor. Dextran sulphate, kappa-carrageenan, pentosan sulphate, polyanethole sulphonate, heparin and fucoidin were shown to compete for the heparin binding site on a fibroblast growth factor at relatively low concentrations (<50 µg/ml). The differential effects of these polyanions in potentiating the biological activities of fibroblast growth factor in relation to their ability to compete for the heparin-binding site of a fibroblast growth factor is discussed. Similarly, Hoover et al (1980) (in Circulation Research 47: 578: 583) studied the in vitro effects of heparin on the growth of rat aortic smooth muscle cells. The results showed that there was a highly specific interaction with regard to molecule and cell type i.e. other polyanions. The suggestion was that heparin and related dextran sulphate could in some way bind to certain factors responsible for cell growth and subsequent proliferation.

Non-enzymic glycosylation of basic fibroblast growth factor has recently been demonstrated to decrease the mitogenic activity of intracellular basic fibroblast growth factor. Loss of this bioactivity has been implicated in impaired wound healing and microangiopathics of diabetes mellitus. In addition to intracellular localisation, basic fibroblast growth factor is widely distributed in the extracellular matrix, primarily bound to heparin sulphate proteoglycans. Nissen et al (1999) measured the effect of non-enzymic glycosylation on basic fibroblast growth factor bound to heparin, heparin sulphate and related compounds (see Biochemical Journal 338: 637-642). When heparin was added to basic fibroblast growth factor prior to non-enzymic glycosylation, the mitogenic activity and heparin affinity of basic fibroblast growth factor were nearly completely preserved.
Heparin sulphate, low molecular mass heparin and the polysaccharide, dextran sulphate, demonstrated a similar protective effect.

The invention is now further described by reference to the following, non-limiting, examples (together with a comparative example).

### Example 1

90 ml of water was added to 50 g of 40,000 molecular weight dextran and manually mixed to encourage the dextran to enter into solution. Afterwards the mixture was placed on a magnetic stirrer and allowed to mix continuously for 15 minutes or until the solution was clear and particle free.

5 g of urea were then added to the solubilised dextran and the mixture placed back on the magnetic stirrer for a further 15 minutes to ensure that the urea had entered into solution with the dextran. Finally, 10 ml of formalin (a 38% (w/v) aqueous solution of formaldehyde hydrate) providing 3.8 g of formaldehyde was added to complete the mixture which was again allowed to stir for 15 minutes. This mixture was the impregnated into knitted polyester grafts using vacuum techniques.

Gels were formed by placing the dextran impregnated grafts in an oven at 150°C for 2 hours. During this time a cross-linking reaction was taking place. Grafts were washed for a minimum of four hours to ensure removal of any residual formaldehyde. Finished grafts were softened by exposure to 100% glycerol for 10 minutes followed by an alcohol wash to remove any excess glycerol. Grafts were then left to air dry.

### Example 2

92 ml of water was added to 40 g of 40,000 molecular weight dextran and manually mixed to encourage the dextran to enter into solution. Afterwards, the mixture was placed on a magnetic stirrer and allowed to mix continuously for 15 minutes or until the solution was clear and particle free.

4 g of urea were then added to the solubilised dextran and the mixture placed back on the magnetic stirrer for a further 15 minutes to ensure that the urea had entered into solution with the dextran. Finally, 8 ml of formalin (38% aqueous solution of formaldehyde hydrate) providing 3.04 g formaldehyde was added to complete the mixture which was again allowed to stir for 15 minutes. Knitted polyester grafts were vacuum impregnated with this mixture.

Gels were formed by placing the grafts in an oven at 50°C for 12 hours. During this time a cross-linking reaction was taking place. Grafts were washed for a minimum of four hours to ensure removal of any residual formaldehyde. Finished grafts were softened by exposure to 80% (v/v, in water) glycerol for 10 minutes followed by an alcohol wash to remove any excess glycerol. Grafts were then left to air dry.

### Example 3 - Preparation of Dextran Blends

**Table 1:**

| Dextran/dextran sulphate crosslinked blends | | | | |
|---|---|---|---|---|
| **Dextran (g)** | **Dextran Sulphate (g)** | **Urea (g)** | **Formaldehyde (ml)** | **Water (ml)** |
| 10 | 0 | 1 | 2 | 18 |
| 9 | 1 | 1 | 2 | 18 |
| 8 | 2 | 1 | 2 | 18 |
| 7 | 3 | 1 | 2 | 18 |
| 6 | 4 | 1 | 2 | 18 |
| 5 | 5 | 1 | 2 | 18 |

Dextran of molecular weigh 40,000 was weighed and the corresponding weight of dextran sulphate of similar molecular weight were added together. The correct level of water was added and the substances mixed thorougly until clear. The urea was mixed again before final addition of formaldehyde. The completed preparation was further mixed to ensure complete solubilisation. Gels were formed when the completed mix was placed in an oven for a specified time period. Samples were then washed for 3 hours in continuous running water.

Corresponding analysis (Dionex ion chromatography) to investigate the presence of sulphate groups in each of the samples showed significant detection of sulphation, with least levels present in sample 1 (1 g of dextran sulphate) and most in sample 5 (5 g of dextran sulphate). It was proposed that the dextran sulphate had become entrapped within the network of cross-linked dextran chains to form an interpenetrating network with the potential to offer corresponding sulphation to the gels for subsequent attachment of growth factors. From the results various sulphanated gels could be prepared, see Examples 4 to 7.

### Example 4

90 ml of water was added to a mixture of 30 g of 40,000 molecular weight dextran and 20 g of 40,000 molecular weight dextran sulphate and manually mixed to encourage the two forms of dextran to enter into solution with each other. Afterwards, the mixture was placed on a magnetic stirrer and allowed to mix continuously for 15 minutes or until the solution was clear and particle free.

5 g of urea was added and the mixture placed back on the magnetic stirrer for a further 15 minutes to ensure that the urea had entered into solution with the two dextran species. Finally, 10 ml of formaldehyde was added to complete the mixture, which was again allowed to stir for 15 minutes.

Gels were formed by placing the dextran mixture into an oven at 50°C for a minimum of 12 hours. During this time, a cross-linking reaction took place. The subsequent dextran mixtures were washed for a minimum of 3 hours under continuous running water.

### Example 5

90 ml of water was added to a mixture of 25 g of 40,000 molecular weight dextran and 25 g of 40,000 molecular weight dextran sulphate and manually mixed to encourage the two forms of dextran to enter into solution with each other. Afterwards the mixture was placed on a magnetic stirrer and allowed to mix continuously for 15 minutes or until the solution was clear and particle free.

5 g of urea was added and the mixture was placed back on the magnetic stirrer for a further 15 minutes to ensure that the urea had entered into solution with the two dextran species. Finally, 10 ml of formaldehyde was added to complete the mixture, which was again allowed to stir for 15 minutes.

Gels were formed by placing the dextran mixture into an oven at 50°C for a minimum of 12 hours. During this time a cross-linking reaction took place. The subsequent dextran mixtures were washed for a minimum of 3 hours under continuous running water.

### Example 6

90 ml of water was added to a mixture of 30 g of 40,000 molecular weight dextran and 20 g of 40,000 molecular weight dextran sulphate and manually mixed to encourage the two forms of dextran to enter into solution with each other. Afterwards the mixture was placed on a magnetic stirrer and allowed to mix continuously for 15 minutes or until the solution was clear and particle free.

5 g of urea was added and the mixture placed back on the magnetic stirrer for a further 15 minutes to ensure that the urea had entered into solution with the two dextran species. Finally, 10 ml of formaldehyde was added to complete the mixture, which was again allowed to stir for 15 minutes.

Gels were formed by placing the dextran mixture into an oven at 100°C for a minimum of 2 hours. During this time a cross-linking reaction took place. The subsequent dextran mixtures were washed for a minimum of 3 hours under continuos running water.

### Example 7

90 ml of water was added to a mixture of 25 g of 40,000 molecular weight dextran and 25 g of 40,000 molecular weight dextran sulphate and manually mixed to encourage the two forms of dextran to enter into solution with each other. Afterwards the mixture was placed on a magnetic stirrer and allowed to mix continuously for 15 minutes or until the solution was clear and particle free.

5 g of urea was added and the mixture placed back on the magnetic stirrer for a further 15 minutes to ensure that the urea had entered into solution with the two dextran species. Finally, 10 ml of formaldehyde was added to complete the mixture, which was again allowed to stir for 15 minutes.

Gels were formed by placing the dextran mixture into an oven at 100°C for a minimum of 2 hours. During this time a cross-linking reaction took place. The subsequent dextran mixtures were washed for a minimum of 3 hours under continuos running water.

### Example 8 - Resorption Rates

The resorption rate of sealant from dextran sealed grafts made according to Examples 1 and 2 were determined *in vitro* by incubating graft samples of known weight in buffer and weighing the grafts again after drying to measure the amount of sealant remaining. Urea formaldehyde cross-linked dextran was found to be hydrolysed at a rate comparable to the gelatin sealant of EP-B-0,183,365.

The hydrolysis profiles of urea-formaldehyde cross-linked dextran and formaldehyde cross-linked gelatin grafts are detailed in Table 2. Hydrolysis was performed at 37°C over a period of up to 4 weeks at 125 rpm.

**Table 2**

| Comparative hydrolysis results for dextran and gelatin coated vascular grafts. The gelatin coated grafts were produced in accordance with Example 1 of EP-B-0,183,365. | | |
|---|---|---|
| Day | % gel degraded | |
| | Dextran | Gelatin* |
| 0 | 0 | 0 |
| 3 | 5 | 30 |
| 6 | 15 | 70 |
| 12 | 25 | 95 |
| 28 | 95 | 100 |

| | | |
|---|---|---|
| *Comparative Example | | |

### Example 9 - Implantation

Grafts prepared according to Example 1 were implanted into the abdominal aorta of dogs for 2 weeks and 4 weeks respectively. Histological examination of the explanted devices showed that the sealant was resorbed as expected within 1 month and that the normal healing process was not adversely affected.

## Claims

1. A bioresorbable sealant composition for coating a prosthetic graft, said composition comprising a polymer obtainable by cross-linking dextran molecules by formaldehyde and urea condensation.

2. The sealant as claimed in Claim 1, wherein said dextran molecules include naturally occurring dextran, hydrophilic hydroxyl group-containing derivatives of dextran or modified forms of dextran containing other reactive groups, for example dextran sulphate.

3. The sealant as claimed in Claim 1, wherein said naturally occurring dextran is provided by fermentation using *Leuconostoc mesenteroides* bacteria.

4. The sealant as claimed in any one of Claims 1 to 3 wherein the dextran molecules have a molecular weight of 30,000 to 60,000.

5. A method of producing a substantially non-porous graft by exposing at least one surface of a flexible material to a mixture of dextran, urea and formaldehyde, and incubating at temperatures of from 20°C to 250°C for a time sufficient for cross-linking of said dextran on said surface to take place.

6. The method as claimed in Claim 5 wherein the temperature is from 30°C to 200°C.

7. The method as claimed in either one of Claims 5 and 6 wherein said flexible material is a polyester knitted or woven fabric, or a PTFE-based material.

8. The method as claimed in Claim 7 wherein said fabric material is expanded PTFE.

9. The method as claimed in any one of Claims 5 to 8 further including the step of practising said cross-linked dextran by exposure of said coated surface to glycerol and, optionally, thereafter removing excess glycerol by alcohol rinsing.

10. A prosthetic graft impregnated or coated with the bioresorbable sealant as claimed in any one of Claims 1 to 4.

11. A method of forming polymerised dextran for use as a biodegradable coating for a prosthetic graft, said method comprising:
a) exposing a water-based solution of dextran to 2 to 25 (weight %) of urea and allowing the urea to enter into solution to form a mixture;
b) exposing the mixture of step a) to formaldehyde;
c) heating the mixture of step b) at temperatures between 20 to 250°C for a time sufficient to allow polymerisation to occur.

12. The method as claimed in Claim 11 wherein 50 to 100% (by weight) of formaldehyde, by reference to the weight of urea, is added.

13. The method as claimed in Claim 12 wherein 70 to 80% (be weight) of formaldehyde, by reference to the weight of urea, is added.

14. The method as claimed in any one of Claims 11 to 13 wherein the temperature is from 30°C to 200°C.

15. The method as claimed in any one of Claims 11 to 14 wherein said dextran has a molecular weight of 30,000 to 60,000.

## Patentansprüche

1. Eine bioresorbierbare Dichtmittel-Zusammensetzung zum Beschichten eines prothetischen Transplantats, wobei die Zusammensetzung ein Polymer beinhaltet, das durch das Vernetzen von Dextranmolekülen durch Formaldehyd und das Kondensieren von Harnstoff gewonnen werden kann.

2. Dichtmittel gemäß Anspruch 1, wobei die Dextranmoleküle natürlich vorkommendes Dextran, hydrophile Hydroxylgruppe enthaltende Dextranderivate oder modifizierte Dextranarten, die andere reaktionsfähige Gruppen, zum Beispiel Dextransulfat, enthalten, umfassen.

3. Dichtmittel gemäß Anspruch 1, wobei das natürlich vorkommende Dextran durch Fermentation unter Verwendung von *Leuconostoc mesenteroides*-Bakterien bereitgestellt ist.

4. Dichtmittel gemäß einem der Ansprüche 1 bis 3, wobei die Dextranmoleküle eine relative Molekülmasse von 30.000 bis 60.000 haben.

5. Ein Verfahren zum Herstellen eines im Wesentlichen nicht porösen Transplantats, indem mindestens eine Fläche eines flexiblen Materials einer Mischung aus Dextran, Harnstoff und Formaldehyd ausgesetzt wird und indem bei Temperaturen von 20 °C bis 250 °C über einen Zeitraum, der für das Vernetzen des Dextrans auf der Fläche ausreichend ist, eine Inkubation geschieht.

6. Verfahren gemäß Anspruch 5, wobei die Temperatur zwischen 30 °C und 200 °C liegt.

7. Verfahren gemäß Anspruch 5 oder Anspruch 6, wobei das flexible Material ein Wirk- oder Webstoff aus Polyester oder ein auf PTFE basierendes Material ist.

8. Verfahren gemäß Anspruch 7, wobei das Stoffmaterial expandiertes PTFE ist.

9. Verfahren gemäß einem der Ansprüche 5 bis 8, das ferner den Schritt des Ausübens des vernetzen Dextrans, indem die beschichtete Fläche Glycerol ausgesetzt wird und wahlweise nachfolgende überschüssiges Glycerol durch Spülen mit Alkohol entfernt wird, umfasst.

10. Ein Prothese-Transplantat, das mit dem bioresorbierbaren Dichtmittel gemäß einem der Ansprüche 1 bis 4 imprägniert oder beschichtet ist.

11. Ein Verfahren zum Bilden von polymerisiertem Dextran zum Verwenden als eine biologisch abbaubare Beschichtung für ein prothetisches Transplantat, wobei das Verfahren Folgendes beinhaltet:
a) eine auf Wasser basierende Lösung aus Dextran 2 bis 25 (Gewicht-%) Harnstoff aussetzen und das Eintreten von Harnstoff in die Lösung ermöglichen, um eine Mischung zu bilden;
b) die Mischung aus Schritt a) Formaldehyd aussetzen;
c) die Mischung aus Schritt b) bei Temperaturen zwischen 20 und 250 °C über einen Zeitraum, der für die Polymerisation ausreichend ist, erhitzen.

12. Verfahren gemäß Anspruch 11, wobei 50 bis 100 % (Gew.-%) Formaldehyd, unter Bezug auf das Gewicht von Harnstoff, zugegeben werden.

13. Verfahren gemäß Anspruch 12, wobei 70 bis 80 % (Gew.-%) Formaldehyd, unter Bezug auf das Gewicht von Harnstoff, zugegeben werden.

14. Verfahren gemäß einem der Ansprüche 11 bis 13, wobei die Temperatur zwischen 30 °C und 200 °C liegt.

15. Verfahren gemäß einem der Ansprüche 11 bis 14, wobei das Dextran eine relative Molekülmasse von 30.000 bis 60.000 hat.

## Revendications

1. Une composition de matériau d'étanchéité biorésorbable pour enduire une greffe prothétique, ladite composition comprenant un polymère pouvant être obtenu par réticulation de molécules de dextrane par condensation de formaldéhyde et d'urée.

2. Le matériau d'étanchéité tel que revendiqué dans la revendication 1, dans lequel lesdites molécules de dextrane comportent du dextrane existant à l'état naturel, des dérivés contenant des groupes hydroxyles hydrophiles de dextrane ou des formes modifiées de dextrane contenant d'autres groupes réactifs, par exemple du sulfate de dextrane.

3. Le matériau d'étanchéité tel que revendiqué dans la revendication 1, dans lequel ledit dextrane existant à l'état naturel est fourni par fermentation à l'aide de bactéries *Leuconostoc mesenteroides.*

4. Le matériau d'étanchéité tel que revendiqué dans n'importe laquelle des revendications 1 à 3 dans lequel la masse moléculaire des molécules de dextrane est de 30 000 à 60 000.

5. Une méthode pour produire une greffe substantiellement non poreuse en exposant au moins une surface d'un matériau flexible à un mélange de dextrane, d'urée et de formaldéhyde, et en incubant à des températures allant de 20 °C à 250 °C pendant une durée suffisante pour que la réticulation dudit dextrane sur ladite surface ait lieu.

6. La méthode telle que revendiquée dans la revendication 5 dans laquelle la température va de 30 °C à 200 °C.

7. La méthode telle que revendiquée dans l'une ou l'autre des revendications 5 et 6 dans laquelle ledit matériau flexible est un tissu de polyester tricoté ou tissé, ou un matériau à base de PTFE.

8. La méthode telle que revendiquée dans la revendication 7 dans laquelle ledit matériau de tissu est du PTFE expansé.

9. La méthode telle que revendiquée dans n'importe laquelle des revendications 5 à 8 comportant de plus l'étape consistant à mettre en oeuvre ledit dextrane réticulé en exposant ladite surface enduite à du glycérol et, facultativement, à enlever par la suite l'excédent de glycérol par rinçage à l'alcool.

10. Une greffe prothétique imprégnée ou enduite du matériau d'étanchéité biorésorbable tel que revendiqué dans n'importe laquelle des revendications 1 à 4.

11. Une méthode pour former du dextrane polymérisé destiné à être utilisé en tant qu'enduit biodégradable pour une greffe prothétique, ladite méthode consistant :
a) à exposer une solution à base d'eau de dextrane à 2 à 25 (% en poids) d'urée et à laisser l'urée passer en solution pour former un mélange ;
b) à exposer le mélange de l'étape a) à du formaldéhyde ;
c) à chauffer le mélange de l'étape b) à des températures comprises entre 20 et 250 °C pendant une durée suffisante pour permettre à la polymérisation d'avoir lieu.

12. La méthode telle que revendiquée dans la revendication 11 dans laquelle il est ajouté 50 à 100 % (en poids) de formaldéhyde, rapporté au poids de l'urée.

13. La méthode telle que revendiquée dans la revendication 12 dans laquelle il est ajouté 70 à 80 % (en poids) de formaldéhyde, rapporté au poids de l'urée.

14. La méthode telle que revendiquée dans n'importe laquelle des revendications 11 à 13 dans laquelle la température va de 30 °C à 200 °C.

15. La méthode telle que revendiquée dans n'importe laquelle des revendications 11 à 14 dans laquelle la masse moléculaire dudit dextrane est de 30 000 à 60 000.
